(19) 

Europäisches Patentamt
European Patent Office
Office européen des brevets

(11) **EP 4 557 300 A1**

(12) **EUROPEAN PATENT APPLICATION**
published in accordance with Art. 153(4) EPC

(43) Date of publication:
**21.05.2025 Bulletin 2025/21**

(21) Application number: **23838431.7**

(22) Date of filing: **09.03.2023**

(51) International Patent Classification (IPC):
*G16B 20/20* (2019.01)    *G16B 40/00* (2019.01)
*G16B 30/00* (2019.01)

(86) International application number:
**PCT/CN2023/080510**

(87) International publication number:
**WO 2024/011929 (18.01.2024 Gazette 2024/03)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **13.07.2022 CN 202210825534**

(71) Applicant: **BGI Genomics Co., Ltd.**
**Yantian District**
**Shenzhen, Guangdong Province 518083 (CN)**

(72) Inventors:
• **YANG, Jiechun**
  **Shenzhen, Guangdong 518083 (CN)**
• **PENG, Jiguang**
  **Shenzhen, Guangdong 518083 (CN)**
• **PENG, Zhiyu**
  **Shenzhen, Guangdong 518083 (CN)**
• **SUN, Juan**
  **Shenzhen, Guangdong 518083 (CN)**
• **XIANG, Jiale**
  **Shenzhen, Guangdong 518083 (CN)**
• **LIU, Jingjuan**
  **Shenzhen, Guangdong 518083 (CN)**
• **LI, Jingrou**
  **Shenzhen, Guangdong 518083 (CN)**

(74) Representative: **Hoffmann Eitle**
**Patent- und Rechtsanwälte PartmbB**
**Arabellastraße 30**
**81925 München (DE)**

(54) **METHOD AND APPARATUS FOR DETECTING FETAL CHROMOSOMAL ANEUPLOIDY, AND STORAGE MEDIUM**

(57)    The present application discloses a method and apparatus for detecting fetal chromosomal aneuploidy, and a storage medium. The method for detecting fetal chromosomal aneuploidy of the present application comprises: according to the concentration of fetal DNA in the cell-free DNA in the blood of a pregnant woman to be tested, a Z value, and chimerism, calculating a new Z value of the sample to be tested, and according to the new Z value, determining whether an aneuploidy abnormality has occurred in fetal chromosomes, the chimerism being the ratio of abnormal fetal cells to all fetal cells. In the present application, chimerism is inventively applied to fetal chromosomal aneuploidy detection, and three variables, i.e., the fetal DNA concentration, the chimerism, and the Z value, are taken into consideration to calculate the new Z value, such that NIPT accuracy can be improved, and true positive and false positive samples can be distinguished well, thereby reducing false positive samples; and the new Z value conforms to a normal distribution, such that the current supervision and clinical use requirements can be met, and data distribution fluctuations are reduced, thereby reducing the gray scale rate and the retest rate, and improving the stability of the test result.

Figure 1

EP 4 557 300 A1

## Description

### Technical Field

The present application relates to the technical field of detecting fetal chromosomal aneuploidy abnormalities, and in particular to a method, device and storage medium for detecting fetal chromosomal aneuploidy abnormalities.

### Background Art

Fetal chromosomal aneuploidy abnormality means that the fetal chromosomes are aneuploid. A normal fetus has 23 pairs of (46) chromosomes, that is, the chromosomes are euploid. If there is chromosome deletion or chromosome increase, forming aneuploidy, it indicates that the fetal chromosomes are abnormal, that is, there is a fetal chromosomal aneuploidy abnormality.

At present, the clinically common fetal chromosomal aneuploidy abnormalities are Down syndrome, Edwards syndrome and Patau syndrome.

Down syndrome (Trisomy 21 syndrome) is a genetic disease caused by trisomy of chromosome 21, and its common symptoms include developmental delay, special facial features, and mild to moderate intellectual disability. Currently, there is no effective therapy for Down syndrome. The quality of life of patients can only be improved through daily care and education. In addition to Down syndrome, clinically common fetal chromosomal aneuploidy abnormalities include Edwards syndrome (Trisomy 18 syndrome) and Patau syndrome (Trisomy 13 syndrome), etc., which all can lead to severe developmental abnormalities in children.

The molecular biological mechanism of Down syndrome is that chromosome 21 does not separate during the generation of germ cells, resulting in three copies of chromosome 21 in the fertilized egg, which in turn leads to a series of abnormalities in molecular and developmental biological processes. Since there is no effective treatment for chromosomal aneuploidy syndromes such as Down syndrome, and no specific behavioral or environmental factors related to their onset have been found, the current main response is to prevent the birth of babies with serious genetic disorders such as Down syndrome through prenatal screening of pregnant women, that is, the corresponding test is carried out when the mother is pregnant, and if the relevant indicators are positive or high risk, the birth of trisomy babies is avoided by terminating the pregnancy.

In traditional screening, trisomy risk is assessed through serological markers, such as AFP, free β-hCG, uE3, and Inhibin-A. Since serological markers are indirect indicators and cannot directly reflect the fetal chromosomal aneuploidy status, their sensitivity and specificity are poor. Around 2010, high-throughput sequencing technology gradually emerged and became popular. Through high-throughput sequencing technology, cell-free DNA (cfDNA) in maternal plasma can be accurately detected and quantified, and then the relative content of the target chromosome can be used to screen for chromosomal abnormalities including trisomy 21 (i.e., NIPT, Non-Invasive Prenatal Testing). In 2015, an article was published in "New England" magazine that 15,841 samples were analyzed through a prospective, multi-center clinical trial, which showed that the performance of NIPT was significantly better than traditional screening, and both of sensitivity and specificity thereof reached 99.9% or more; as a contrast, a traditional serological screening method showed a sensitivity of only 78.9%, and a specificity of only 94.6%, proving that NIPT had greatly improved the screening effect of chromosomal aneuploidy syndromes represented by Down syndrome.

However, the performance of NIPT test still needs to be improved. According to the article published by Zhang et al. in 2015, the author analyzed the NIPT test results of 112,669 cases with follow-up results and found that the test performance of traditional NIPT mainly had the following two problems: First, the positive predictive value (PPV) should be improved. According to the data in the article, the positive predictive value of T21 was 92.2%, while the positive predictive value of T18 was 76.6%, and the positive predictive value of T13 was only 32.8%, showing that the traditional NIPT test method had more false positive results and the positive predictive value should be improved. Second, the retest rate was high. According to the data in the article, among the 112,669 samples, there were 3,213 repeated blood drawings, that was, the blood redrawing rate was 2.8%. Blood redrawing means that the first NIPT test value is in the gray area, so it cannot give a negative or positive test result, and a tube of blood needs to be drawn and tested again. In this case, pregnant women not only endure the pain of an extra blood drawing; more importantly, the period for reporting NIPT test results is extended, which may cause pregnant women to miss the best intervention period and bring major hidden dangers to the life and health of pregnant women.

Therefore, how to improve the positive predictive value of NIPT test and reduce the retest rate is the focus and difficulty of research on fetal chromosomal aneuploidy abnormalities.

### Contents of the Invention

The object of the present application is to provide an improved method, device and storage medium for detecting fetal chromosomal aneuploidy abnormalities.

In order to achieve the above purpose, the present application adopts the following technical solutions:

The first aspect of the present application discloses a method for detecting fetal chromosomal aneuploidy abnormality, which comprises obtaining a new Z value, which is marked as $Z_{new}$, by calculation based on fetal DNA concentration, Z value and mosaicism in cell-free DNA of a pregnant woman blood sample to be tested, and determining whether the fetal chromosome of the sample to be tested has an aneuploidy abnormality; wherein the mosaicism is a ratio of abnormal fetal cells to all fetal cells.

It should be noted that the key to the method for detecting fetal chromosomal aneuploidy abnormality according to the present application is to obtain the new Z value (i.e., $Z_{new}$, which is commonly used and recognized in the field) by calculation based on three variables: fetal DNA concentration, mosaicism (which is an unique indicator of the present application), and traditional Z value. Wherein, the "traditional Z value" refers to the Z value obtained according to the traditional conventional method; the "new Z value" of the present application refers to the Z value obtained by calculation through three variable calculations in the present application. In the method of the present application, the mosaicism is used as an input variable to calculate the "new Z value", which helps to improve the accuracy of NIPT detection, has good discrimination between true positive and false positive samples, and reduces false positive results; the new Z value conforms to the normal distribution, which not only meets the current regulatory and clinical practices requirements, but also greatly reduces the volatility of data distribution, thereby reducing the gray area rate, reducing the retest rate, and improving the stability of test results.

In one embodiment of the present application, the new Z value of the sample to be tested is calculated based on the fetal DNA concentration, Z value and mosaicism in cell-free DNA of the pregnant women blood sample to be tested, which comprises: inputting the fetal DNA concentration, Z value and mosaicism into a fetal chromosomal aneuploidy abnormality detection model to obtain a model output value corresponding to the sample to be tested, and obtaining the new Z value of the sample to be tested by mapping the model output value; wherein the fetal chromosomal aneuploidy abnormality detection model is a model obtained by using several samples with known fetal chromosome conditions as training samples, the training samples including positive samples and negative samples of fetal chromosomal aneuploidy abnormalities, and using the fetal DNA concentration, Z value and mosaicism as inputs to train a machine learning model to obtain a model output value to characterize the fetal chromosome condition based on the integration of three variables of fetal DNA concentration, Z value and mosaicism.

It can be understood that the model training by machine learning to obtain the new Z value is only one embodiment of the present application. It is not excluded that other calculation methods can be used to obtain the new Z value of the present application based on fetal DNA concentration, Z value, and mosaicism.

In one embodiment of the present application, the new Z value of the sample to be tested is obtained by mapping the model output value, which comprises: obtaining the new Z value of the sample to be tested by calculation based on the model output value of the sample to be tested, positive threshold, negative threshold, and median of model output values of all negative samples; wherein the positive threshold is a threshold of model output value corresponding to positive samples, and the negative threshold is a threshold of model output value corresponding to negative samples.

It should be noted that the model output value, also called as machine learning model generated value, is a value outputted by the fetal chromosomal aneuploidy abnormality detection model to evaluate fetal chromosomal aneuploidy abnormality. Unlike the traditional Z value, the threshold of this value cannot be determined based on statistical significance, but can only be determined based on the characteristics of training data; for example, a negative threshold can be determined so that all true positive samples in the training data would not be judged as negative, ensuring that the model would not produce false negatives; a positive threshold can be determined so that as many true positive samples as possible can be judged as positive, and at the same time as few original false positive samples as possible can be judged as positive, thereby reducing false positives and improving the performance of NIPT detection; there is a gray area between the positive threshold and the negative threshold; in order to enable the model output value of the sample to be tested to be directly used to determine the fetal chromosomal aneuploidy abnormality status, the present application further maps the model output value into a new Z value, that is, $Z_{new}$; and, the test results show that the new Z value obtained through the mapping of the present application conforms to the normal distribution, and the center of the distribution is located at 0. Therefore, Z>3 can still be used as the positive judgment standard, and Z<1.96 can be used as the negative judgment standard.

In one embodiment of the present application, the median of the model output values of negative samples is a median of the model output values of all negative samples obtained by re-inputting all negative training samples into the fetal chromosomal aneuploidy abnormality detection model.

In one embodiment of the present application, the new Z value of the sample to be tested is obtained by mapping the model output value, which comprises the following mapping methods:

when the model output value of the sample to be tested is greater than the positive threshold,

$$Z_{new} = LD - cut_p + 3;$$

when the model output value of the sample to be tested is less than the positive threshold and greater than the negative threshold,

$$Z_{new} = \frac{LD - cut_n}{cut_p - cut_n} * 1.04 + 1.96 \quad ;$$

when the model output value of the sample to be tested is less than the negative threshold,

$$Z_{new} = \frac{LD - Med}{cut_n - Med} * 1.96 \quad ;$$

in the above formula, $Z_{new}$ is the new Z value, $LD$ is the model output value of the sample to be tested, $cut_p$ is the positive threshold, $cut_n$ is the negative threshold, and $Med$ is the median of the model output values of all negative samples.

It should be noted that in the present application, the concept of obtaining the new Z value by mapping the model output value is as follows:

1) when the model output value is greater than the positive threshold, the new Z value obtained by the final conversion needs to be greater than 3, because the clinical practice of judging trisomy positive takes Z>3 as threshold.

2) when the model output value is in the gray area, the new Z value obtained by the final conversion needs to be between 1.96 and 3, because the clinical practice takes Z~[1.96,3) as range of the gray area.

3) when the model output value is less than the negative threshold, the new Z value obtained by the final conversion needs to be less than 1.96, because the clinical practice of judging trisomy negative takes Z < 1.96 as threshold.

In addition, the present application also ensures that the median of new Z value of the negative samples is 0, because for the standard normal distribution, the median should be equal to 0; therefore, $Med$ is considered in the formula, that is, the median of model output value of the negative samples. According to the above mapping formula, it can be found that when the model output value is equal to the median of model output value of the negative samples, the new Z value is 0.
It should also be noted that the specific values in the above formula are the data specifically obtained in an embodiment of the present application; it can be understood that if the training samples change, the data in the corresponding mapping formula will also change; however, the basic principle of obtaining the new Z value by mapping formula remains unchanged.
In one embodiment of the present application, it is based on the new Z value to determine whether aneuploidy abnormality occurs in the fetal chromosome of the sample to be tested, which comprises: it is judged as positive, that is, fetal chromosomal aneuploidy abnormality occurs, when the new Z value is greater than 3; and it is judged as negative, that is, the fetal chromosomes are normal, when the new Z value is less than 1.96.
In one embodiment of the present application, the machine learning model is a linear discriminant analysis model (linear discriminant analysis, abbreviated as LDA).
In one embodiment of the present application, fetal abnormal cells are cells with fetal chromosomal aneuploidy abnormality.
In one embodiment of the present application, the fetal DNA concentration and Z value in the pregnant woman blood cell-free DNA are calculated and obtained through high-throughput sequencing data of the pregnant woman blood cell-free DNA.
In one embodiment of the present application, the mosaicism is calculated by Formula I;

Formula I: $$Mosaic_k = \frac{fra_k}{FF}$$

in Formula I, $Mosaic_k$ is the mosaicism of chromosome $k$, $fra_k$ is the relative fetal concentration of chromosome $k$, and *FF* is the fetal DNA concentration;

*fra$_k$* is calculated using Formula II;

$$\text{Formula II:} \quad fra_k = \frac{\overline{UR}_k - \overline{UR}_{auto}}{\overline{UR}_{auto}} * 2$$

in Formula II, *fra$_k$* is the relative fetal concentration of chromosome *k*, $\overline{UR}_k$ is the average of corrected depths of chromosome *k*, and $\overline{UR}_{auto}$ is the average of corrected depths of all autosomal chromosomes;

in Formula I and Formula II, the value of k ranges from 1 to 22;

when *Mosaic$_k$* is 0, it indicates that the chromosome *k* of fetus is normal; when *Mosaic$_k$* is 1, it indicates that the chromosome *k* of fetus is completely trisomic; when *Mosaic$_k$* is between 0 and 1, it indicates that the chromosome *k* of fetus is mosaic. In the present application, the existence of mosaicism in fetal chromosome *k* means that the chromosome *k* in some fetal cells is in a trisomic state, and the chromosome *k* in some other fetal cells is in a non-trisomic state; in principle, under the condition of fixed fetal DNA concentration, if the fetus is in a complete trisomic state, the trisomy signal in peripheral blood of pregnant woman is strong; if the fetus is in a mosaic trisomic state, the trisomy signal in peripheral blood of pregnant woman is weak; and when the mosaicism is low, it is generally false positive caused by data fluctuations, and when the mosaicism is high, it is generally true positive.

In one embodiment of the present application, the average value of corrected depth of each chromosome and the average value of corrected depth of all autosomal chromosomes are calculated and obtained through high-throughput sequencing data of the pregnant women blood cell-free DNA.

In one embodiment of the present application, the method for detecting fetal chromosomal aneuploidy abnormality comprises the following steps:

step of data acquisition, which comprises obtaining high-throughput sequencing data of the pregnant women blood cell-free DNA to be tested;

step of data processing, which comprises calculating fetal DNA concentration and Z value based on the high-throughput sequencing data of the pregnant women blood cell-free DNA to be tested;

step of mosaicism calculation, which comprises calculating the mosaicism of each chromosome according to Formula I;

step of new Z value, which comprises calculating and obtaining a new Z value of the sample to be tested based on the fetal DNA concentration, Z value, and mosaicism in the pregnant women blood cell-free DNA to be tested;

step of determining fetal chromosomal aneuploidy abnormality, which comprises determining on the basis of the new Z value whether chromosomal aneuploidy abnormality occurs in chromosomes of fetus to be tested.

It should be noted that the key to the method for detecting fetal chromosomal aneuploidy abnormality according to the present application is to obtain the model output value through the fetal chromosomal aneuploidy abnormality detection model based on comprehensive consideration of three variables: fetal DNA concentration, mosaicism and traditional Z value, and converting the value into the new Z value (i.e., $Z_{new}$, which is commonly used and recognized in the field). Wherein, the "traditional Z value" refers to the Z value obtained by the "data processing step" according to the traditional conventional method, and in order to better distinguish the "new Z value" of the present application, the Z value obtained by the "data processing step" is called "traditional Z value", while the Z value obtained by the present application through model output value mapping is called "new Z value". In the present application, the incorporation of mosaicism into the machine learning model helps to improve the accuracy of NIPT detection, has good discrimination between true positive and false positive samples, and reduces false positive results; the new Z value conforms to the normal distribution, which not only meets the current regulatory and clinical practices requirements, but also greatly reduces the volatility of data distribution, thereby reducing the gray area rate, reducing the retest rate, and improving the stability of test results.

The second aspect of the present application discloses a method for constructing a fetal chromosomal aneuploidy abnormality detection model, which comprises: using several samples with known fetal chromosome conditions are used as training samples, the training samples including positive samples and negative samples of fetal chromosomal aneuploidy abnormalities, inputting fetal DNA concentration, Z value and mosaicism to train a machine learning model to obtain a model output value for characterizing fetal chromosome condition based on the integration of three variables of fetal DNA concentration, Z value and mosaicism, thereby obtaining a mode, that is, the fetal chromosomal aneuploidy abnormality detection model.

It should be noted that the method for constructing a fetal chromosomal aneuploidy abnormality detection model of the present application is actually a method for constructing a fetal chromosomal aneuploidy abnormality detection model in the method of detecting fetal chromosomal aneuploidy abnormality of the present application. Therefore, the methods for calculation of fetal DNA concentration, Z value and mosaicism can refer to the methods of detecting fetal chromosomal aneuploidy abnormality of the present application, so they will not be described again herein.

The third aspect of the present application discloses a device for detecting fetal chromosomal aneuploidy abnormality, which comprises a new Z-value calculation module and a fetal chromosomal aneuploidy abnormality judgment module; the new Z-value calculation module is configured to calculate a new Z value of a sample to be tested based on the fetal DNA concentration, Z value, and mosaicism in a pregnant woman blood cell-free DNA of the sample to be tested, in which the mosaicism is a ratio of abnormal fetal cells to all fetal cells; and the fetal chromosomal aneuploidy abnormality judgment module is configured to determine whether aneuploidy abnormality occurs in the fetal chromosomes of the sample to be tested on the basis of the new Z value.

In one embodiment of the present application, the new Z value calculation module is further configured to input the fetal DNA concentration, Z value and mosaicism into a fetal chromosomal aneuploidy abnormality detection model to obtain a model output value corresponding to the sample to be tested, and to obtain the new Z value of the sample to be tested by mapping the model output value; wherein the fetal chromosomal aneuploidy abnormality detection model is a model obtained by using several samples with known fetal chromosome conditions as training samples, the training samples including positive samples and negative samples of fetal chromosomal aneuploidy abnormalities, and using the fetal DNA concentration, Z value and mosaicism as inputs to train a machine learning model; the model output value is used to characterize the fetal chromosomal condition based on the integration of three variables of fetal DNA concentration, Z value and mosaicism.

Therefore, in one embodiment of the present application, the device of the present application further comprises a model training module, in which several samples of known fetal chromosome conditions are used as training samples, the training samples include positive samples and negative samples of fetal chromosomal aneuploidy abnormalities, the fetal DNA concentration, Z value and mosaicism are used as inputs to train a machine learning model, and a model output value for characterizing fetal chromosomal aneuploidy condition is obtained based on the integration of three variables of fetal DNA concentration, Z value and mosaicism, and thus a model is obtained, that is, the fetal chromosomal aneuploidy abnormality detection model. Preferably, the machine learning model is a linear discriminant analysis model.

In an embodiment of the present application, the new Z value calculation module comprises a model output value analysis sub-module and a Z value mapping sub-module; the model output value analysis sub-module is configured to input the fetal DNA concentration, Z value and mosaicism of the sample to be tested into the fetal chromosomal aneuploidy abnormality detection model to obtain the model output value corresponding to the sample to be tested; the Z-value mapping sub-module is configured to obtain the new Z value of the sample to be tested by calculation based on the model output value of the sample to be tested, as well as positive threshold, negative threshold, and the median of model output values of all negative samples; the positive threshold is a threshold of model output values corresponding to positive samples, and the negative threshold is a threshold of model output values corresponding to negative samples.

In one embodiment of the present application, the Z value mapping sub-module is used to obtain the new Z value according to the following method:

when the model output value of the sample to be tested is greater than the positive threshold,

$$Z_{new} = LD - cut_p + 3;$$

when the model output value of the sample to be tested is less than the positive threshold and greater than the negative threshold,

$$Z_{new} = \frac{LD - cut_n}{cut_p - cut_n} * 1.04 + 1.96;$$

when the model output value of the sample to be tested is less than the negative threshold,

$$Z_{new} = \frac{LD - Med}{cut_n - Med} * 1.96 \; ;$$

in the above formula, $Z_{new}$ is the new Z value, $LD$ is the model output value of the sample to be tested, $cut_p$ is the positive threshold, $cut_n$ is the negative threshold, and $Med$ is the median of model output values of all negative samples.

In one embodiment of the present application, the fetal chromosomal aneuploidy abnormality module is configured to determine whether aneuploidy abnormality occurs in the fetal chromosome of the sample to be tested based on the new Z value, in which when the new Z value is greater than 3, it is judged as positive, that is, fetal chromosomal aneuploidy abnormality occurs; and when the new Z value is less than 1.96, it is judged as negative, that is, the fetal chromosomes are normal.

It should be noted that in the device of the present application, the model training module can be used according to needs, for example, in the case that the fetal chromosomal aneuploidy abnormality detection model, the positive threshold, the negative threshold and the median of model output values of all negative samples have been obtained, other modules can directly call the model and data; therefore, it is not necessary to run the model training module for every detection. Of course, if the training samples change, for example when training samples are added, it is recommended to run the model training module to further improve the model and various data.

It should also be noted that the device for detecting fetal chromosomal aneuploidy abnormality in the present application actually implements the method of detecting fetal chromosomal aneuploidy abnormality in the present application through the modules; therefore, the specific limitations of each module can refer to the method of detecting fetal chromosomal aneuploidy abnormality in the present application. For example, the calculation of fetal DNA concentration, Z value and mosaicism, the specific $Z_{new}$ calculation method, the linear discriminant analysis model, and how to judge positive and negative based on $Z_{new}$, etc., can all refer to the method of detecting fetal chromosomal aneuploidy abnormality in the present application.

The fourth aspect of the present application discloses a device for detecting fetal chromosomal aneuploidy abnormality. The device comprises a memory and a processor; the memory comprises storing a program; the processor comprises executing the program stored in the memory to implement the method of detecting fetal chromosomal aneuploidy abnormality of the present invention or the method for constructing fetal chromosomal aneuploidy abnormality detection model of the present invention.

It can be understood that when the device of the present application implements the method for constructing fetal chromosomal aneuploidy abnormality detection model of the present application by executing the program stored in the memory, the device of the present application is actually a device for model construction, and the model obtained by the construction using the device can be used to detect fetal chromosomal aneuploidy abnormality according to the method of the present application.

The fifth aspect of the present application discloses a computer-readable storage medium, in which a program is stored in the storage medium, and the program can be executed by a processor to implement the method of detecting fetal chromosomal aneuploidy abnormality of the present application or the method for constructing fetal chromosomal aneuploidy abnormality detection model of the present application.

It can be understood that when the program stored in the computer-readable storage medium of the present application can be executed by the processor to implement the method for constructing fetal chromosomal aneuploidy abnormality detection model of the present application, the computer-readable storage medium of the present application actually is a computer-readable storage medium used for model construction. The computer-readable storage medium can be used directly to implement the construction of fetal chromosomal aneuploidy abnormality detection model, and the model obtained thereby can be used to detect fetal chromosomal aneuploidy abnormality according to the method of the present application.

Due to the adoption of the above technical solutions, the beneficial effects of the present application are as follows:

The method and device for detecting fetal chromosomal aneuploidy abnormality of the present application is the first to incorporate mosaicism into the detection of fetal chromosomal aneuploidy abnormality, and to calculate a new Z value based on comprehensive consideration of three variables: fetal DNA concentration, mosaicism and traditional Z value. The method and device of the present application can improve the accuracy of NIPT detection, have good discrimination between true positive and false positive samples, and reduce false positives. Moreover, the new Z value conforms to the normal distribution, which not only meets the current regulatory and clinical practices requirements, but also reduces the volatility of data distribution, thereby reducing the gray area rate, reducing the retest rate, and improving the stability of test results.

## Brief Description of the Drawings

Figure 1 shows a flow chart of a method for detecting fetal chromosomal aneuploidy abnormality in an example of the present application;

Figure 2 shows a structural block diagram of a device for detecting fetal chromosomal aneuploidy abnormality in an example of the present application;

Figure 3 shows an analysis chart of T13 mosaicism of 10,240 samples in an example of the present application;

Figure 4 shows a Q-Q diagram of the new Z value of chromosome 21 of 10,000 samples in an example of the present application;

Figure 5 shows a distribution diagram of the traditional Z value and the new Z value of chromosome 13 of 10,000 samples in an example of the present application.

## Specific Models for Carrying Out the Invention

The present application will be further described in detail below through specific examples in conjunction with the accompanying drawings. In the following examples, many details are described in order to make the present application be better understood. However, those skilled in the art can easily realize that some of the features may be omitted in different situations, or may be replaced by other devices, materials, and methods. In some cases, some operations related to the present application are not shown or described in the description in order to avoid the core part of the present application being overwhelmed by excessive descriptions. For those skilled in the art, it is unnecessary to describe these relevant operations in detail, and the relevant operations can be fully understood according to the description in the description and the general technical knowledge in the field.

In the present application, mosaicism is creatively used as a variable in calculating the new Z value to improve the accuracy of NIPT detection. Therefore, the present application proposes a method for detecting fetal chromosomal aneuploidy abnormality, which comprises obtaining a new Z value of the sample to be tested by calculation based on the fetal DNA concentration, Z value, and mosaicism in the pregnant woman blood cell-free DNA of the sample to be tested, and determining whether aneuploidy abnormality occurs in the fetal chromosomes of the sample to be tested based on the new Z value; wherein the mosaicism is a ratio of abnormal fetal cells to all fetal cells.

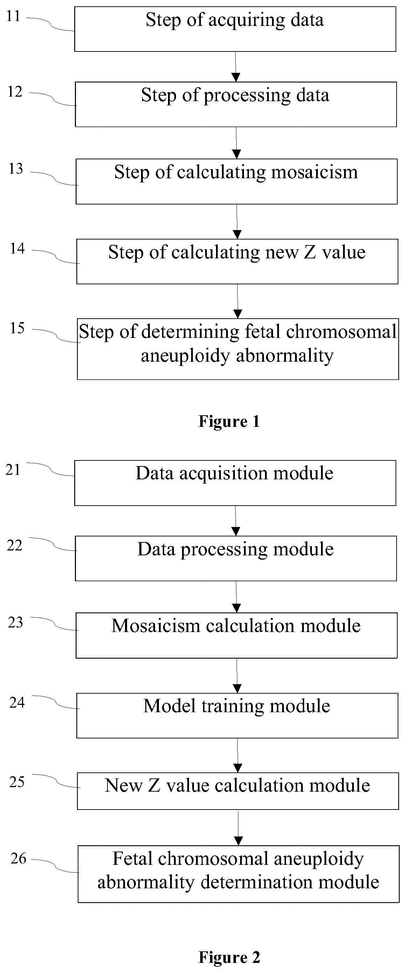

In one embodiment of the present application, the method for detecting fetal chromosomal aneuploidy abnormality is shown in Figure 1 and specifically comprises a data acquisition step **11,** a data processing step **12,** a mosaicism calculation step **13,** a new Z value calculation step **14,** and a fetal chromosomal aneuploidy abnormality determination step **15.**

Wherein, the data acquisition step **11** comprises acquiring high-throughput sequencing data of pregnant women blood cell-free DNA to be tested. For example, in one embodiment of the present application, the downloaded data is a fastq format file generated by the sequencer.

The data processing step **12** comprises calculating fetal DNA concentration, Z value, average of corrected depths of each chromosome, and average of corrected depths of all autosomal chromosomes based on the obtained high-throughput sequencing data of pregnant women blood cell-free DNA to be tested. In one embodiment of the present application, this step comprises common operations of the conventional NIPT process, which specifically comprises the following:

A) Sequence alignment and filtering: The sequence information contained in the fastq format file generated by the sequencer is aligned to a human reference genome, such as GRCh37/hg19, through public software, such as BWA (0.7.7-r441), and filtering is performed to remove sequences with poor alignment quality, multiple alignment sequences, repeated sequences, and imperfect alignment sequences, and remain unique alignment sequences, and the coordinates and other information of each unique alignment sequence are stored in BAM format files.

B) Window division and data correction: The human reference genome is divided into windows of about 60kb, and the number of uniquely aligned sequences in each 60kb window is recorded as the original depth information of the window, that is, the window depth. Furthermore, GC correction and inter-sample correction are performed on the original depth of each window to obtain the corrected depth information (i.e. UR) of each window. The corrected depths of all windows on a chromosome are averaged to obtain the "average of corrected depths for chromosome k". In the present application, the averages of corrected depths for all autosomal chromosomes are calculated.

C) Calculation of fetal DNA concentration: In the present application, different calculation methods are used for male fetuses and female fetuses, which are specifically as follows:

The male fetal concentration is calculated as follows:

The fetal concentration of male fetus is determined by the proportion of Y chromosome, in which the mean window UR of Y chromosome is divided by the mean UR of autosomal chromosomes, and then multiplied by 2 to obtain the fetal concentration *FF* of male fetus:

$$FF = \frac{2 \times \overline{UR}_{chrY}}{\overline{UR}_{autosomes}}$$

The female fetal concentration is calculated as follows:

The fetal concentration of female fetus is estimated by establishing a high-dimensional regression model using the non-uniform distribution of fetal cell-free DNA on the genome, in which the background assumption lies in the differences between distribution characteristics of fetal cfDNA and maternal cfDNA on the genome of fetus, regardless of male or female fetus. Therefore, the fetal concentration estimated by the Y chromosome method of male fetus is used as the input of training model, and the regression model is constructed using the neural network machine learning method, which is specifically as follows:

$$z_j^l = f\left(\sum_k w_{jk}^l z_k^{l-1} + b_j^l\right)$$

wherein *l* is the sequence number of network layer, the first layer is an input layer, the last layer is an output layer (only one neuron), and the middle is a hidden layer. $z_j^l$ the value of the j-th neuron in the *l*-th layer, $z_k^{l-1}$ is the value of the k-th neuron in the (*l*-1)-th layer, $w_{jk}^l$ is the connection weight from the k-th neuron in the (*l*-1)-th layer to the j-th neuron in the *l*-th layer, $b_j^l$ is the input bias of the j-th neuron in the *l*-th layer. The most commonly used form of function *f* is rectified linear unit, that is, *f*(x)=*max*(0,*x*). *w* and *b* are obtained when training the model. When applying the model, the values of neurons are calculated layer by layer according to the above formula. The value of neurons in the last layer is the predicted value of the fetal concentration model.

D) Calculation of traditional Z value: The depths of all intervals on a certain chromosome conform to the normal distribution. Therefore, using a certain chromosome as a reference, the Z value of a chromosome to be tested can be calculated using the distribution of depths of intervals of the chromosome to be tested. The Z value can be used as a basis to determine whether the chromosome is trisomic.

Specifically, in the present application, the traditional Z value calculation method is as follows:

The corrected depth information UR of each window of autosomal chromosomes conforms to the Poisson distribution. When the number of windows is large, it conforms to the normal distribution. For normal samples, there is no significant difference between the distribution of UR of the chromosome to be tested and the distribution of UR of the reference chromosome, while there is slight difference for abnormal samples. The Z test can be used to determine whether fetal chromosomal aneuploidy abnormality occurs, which is specifically as follows:

$$Z_i = \text{mean}\left(\frac{\overline{UR}_i - \overline{UR}_j}{\sqrt{\frac{SD_i^2}{L_i} + \frac{SD_j^2}{L_j}}}\right)$$

(j=1...22, j≠i, two values before and after it are removed after sorting)
wherein:

$\overline{UR_i}$: mean value of UR of chromosome $i$;

$\overline{UR_j}$: mean value of UR of chromosome $j$;

$SD_i$: represents the standard deviation of UR of chromosome $i$;

$SD_j$: represents the standard deviation of UR of chromosome $j$;

$L_i$: represents the number of windows divided for chromosome $i$;

$L_j$: represents the number of windows divided for chromosome $j$;

$Z_i$: represents the significance of aneuploidy of chromosome i, indicating the difference from euploidy.

The above formula involves the comparison of 22 autosomal chromosomes within the same sample between each other. The background assumption of this is that most of the chromosomes in a sample should be normal diploid. Therefore, when a target chromosome is compared with the remaining 21 chromosomes 21 times, if the target chromosome is a normal diploid, most of the values of 21 Z tests should be close to 0, and the average thereof will give a negative Z value; on the other hand, if the target chromosome is a trisomy, most of the values of the 21 Z tests should be much greater than 0, and the average thereof will give a positive Z value.

The mosaicism calculation step 13 comprises calculating the mosaicism of each chromosome based on the fetal DNA concentration.

For example, the mosaicism of each chromosome is calculated according to Formula I:

$$\text{Formula I:} \quad Mosaic_k = \frac{fra_k}{FF}$$

in Formula I, $Mosaic_k$ is the mosaicism of chromosome $k$, $fra_k$ is the relative fetal concentration of chromosome $k$, $FF$ is the fetal DNA concentration; $fra_k$ is calculated using Formula II:

$$\text{Formula II:} \quad fra_k = \frac{\overline{UR_k} - \overline{UR_{auto}}}{\overline{UR_{auto}}} * 2$$

in Formula II, $fra_k$ is the relative fetal concentration of chromosome $k$, $\overline{UR_k}$ is the average of corrected depths of chromosome $k$, and $\overline{UR_{auto}}$ is the average of corrected depths of all autosomal chromosomes; in Formula I and Formula II, $k$ is a value between 1 and 22.

When $Mosaic_k$ is 0, it indicates that the chromosome $k$ is normal; when $Mosaic_k$ is 1, it indicates that the fetus's chromosome $k$ is completely trisomic; and when $Mosaic_k$ is between 0 and 1, it indicates that the fetus's chromosome $k$ is mosaic.

It should be noted that the calculation of mosaicism and its inclusion for determining fetal chromosomal aneuploidy abnormality are one of the innovative improvements of the present application. Research shows that not all fetal trisomies are complete trisomies, in other words, not every fetal cell is in a trisomic state, that is, some of fetal cells are in a trisomic state and some are in a non-trisomic state, and this situation is called mosaicism. Fetal mosaicism will affect NIPT detection results. For example, in the case that the fetal DNA concentration is fixed, if the fetus is a complete trisomy, the trisomic signal in peripheral blood of pregnant woman should be stronger; if the fetus is a chimeric trisomy, the trisomic signal in peripheral blood of pregnant women should be weaker. Since NIPT involves multiple steps such as plasma collection, storage, transportation, cfDNA isolation, library construction, and sequencing, slight fluctuations in any step will cause fluctuations in the final detection results. For negative samples, data fluctuations may bring about a weak trisomic signal similar to that of low mosaicism. Therefore, in the present application, it is creatively proposed to quantitatively describe the degree of mosaicism, and further clarify the difference between the mosaicism of true positive samples and the mosaicism of weak trisomic signals caused by data fluctuations, so as to better distinguish the true positive and false positive samples.

The new Z value calculation step **14** comprises obtaining a new Z value of the sample to be tested by calculation based on the fetal DNA concentration, Z value, and mosaicism in the pregnant woman blood cell-free DNA of the sample to be tested; in which, the mosaicism is a ratio of abnormal fetal cells to all fetal cells.

For example, the new Z value calculation step **14** is divided into a model output value analysis sub-step and a Z value mapping sub-step.

The model output value analysis sub-step comprises: inputting the fetal DNA concentration, traditional Z value and mosaicism of the sample to be tested into the fetal chromosomal aneuploidy abnormality detection model to obtain the model output value corresponding to the sample to be tested, in which the fetal chromosomal aneuploidy abnormality detection model is a model obtained by using several samples with known fetal chromosomal aneuploidy abnormality as training samples, using fetal DNA concentration, traditional Z value and mosaicism as inputs, and using model output values as outputs to train a machine learning model.

It should be noted that the machine learning model training is another innovative improvement of the present application. Before training the model, it was found in the researches of the present application that there was a very good linear relationship between the three variables: fetal DNA concentration, mosaicism, and traditional Z value. Therefore, the three variables of fetal DNA concentration, mosaicism, and traditional Z value were put into the LDA (linear discriminant analysis) model for model training, and the trained model was obtained, which was the fetal chromosomal aneuploidy abnormality detection model.

The general form of the LDA model is as follows:

$$LD = w_1 a_1 + w_2 a_2 + \cdots + w_k a_k$$

wherein, $w_k$ is a coefficient, which is the model output value obtained by the model training, while $a_k$ is a variable, which is the sample information input into the model, and in this case, it is the fetal concentration, traditional Z value and mosaicism. Therefore, the coefficients of the three variables of fetal concentration, traditional Z value and mosaicism are actually obtained after the model training. With these three coefficients, in addition to the fetal concentration, traditional Z value and mosaicism of the sample, the result of the machine learning model, that is, the model output value (*LD* value) can be obtained through the above formula.

The Z value mapping sub-step comprises obtaining a new Z value of the sample to be tested by calculation based on the model output value of the sample to be tested, the positive threshold, the negative threshold, and the median of model output values of all negative samples, which is marked as $Z_{new}$.

It should be noted that the results generated by the machine learning model no longer conform to a statistically significant distribution, so the threshold cannot be determined based on statistical significance like that for the traditional Z value, and the threshold can only be determined based on the characteristics of the training data. The negative threshold is determined so that all true positive samples in the training data cannot be judged as negative, ensuring that the model will not generate false negatives. The positive threshold is determined so that as many true positive samples as possible can be judged as positive, while as few original false positive samples as possible can be judged as positive, thereby reducing false positives and improving the performance of NIPT detection. The gray area is between the positive threshold and the negative threshold.

The results generated by the machine learning model no longer conform to a statistically significant distribution. However, in actual clinical practices, according to clinical usage habits and regulatory requirements, NIPT trisomy detection results must give feedback in the form of Z value, and take 3 as the positive threshold. Thus, how to transform the non-statistically significant results of the machine learning model into a statistically significant Z value is the third innovative improvement of the present application. The machine learning model used in one embodiment of the present application is a linear model, so that the final results generated by the machine learning model can maintain the distribution characteristics of the traditional Z value. Therefore, in the present application, the mapping method is creatively used to map the model output value into a new Z value, which can not only improve the performance of NIPT detection, but also make the final results to have distribution characteristics similar to that of the Z value, that is, it conforms to the normal distribution with the center at 0.

In one embodiment of the present application, the specific mapping method is as follows:

when the model output value of the sample to be tested is greater than the positive threshold,

$$Z_{new} = LD - cut_p + 3;$$

when the model output value of the sample to be tested is less than the positive threshold and greater than the negative threshold,

$$Z_{new} = \frac{LD - cut_n}{cut_p - cut_n} * 1.04 + 1.96 \quad ;$$

when the model output value of the sample to be tested is less than the negative threshold,

$$Z_{new} = \frac{LD - Med}{cut_n - Med} * 1.96 \quad ;$$

in the above formula, $Z_{new}$ is the new Z value, $LD$ is the model output value of the sample to be tested, $cut_p$ is the positive threshold, $cut_n$ is the negative threshold, and $Med$ is the median of the model output values of all negative samples.

The fetal chromosomal aneuploidy abnormality determination step **15** comprises determining whether aneuploidy abnormality occurs in chromosomes of the fetus to be tested based on the new Z value.

In one embodiment of the present application, the new Z value obtained through the segmented mapping also conforms to the normal distribution, and the center of distribution is located at 0; therefore, Z>3 can still be used as the positive judgment value, and Z<1.96 can still be used as the negative judgment value.

Based on the method of detecting fetal chromosomal aneuploidy abnormality in the present application, the present application proposes a method for constructing a fetal chromosomal aneuploidy abnormality detection model, which comprises: using several samples with known fetal chromosomal conditions as training samples (in which the training samples include positive and negative samples of fetal chromosomal aneuploidy abnormalities), using fetal DNA concentration, Z value and mosaicism as inputs to train a machine learning model to obtain a model output value characterizing the fetal chromosome condition based on the fetal DNA concentration, Z value and mosaicism, thereby obtaining a model, that is, the fetal chromosomal aneuploidy abnormality detection model. Wherein, the calculation methods of fetal DNA concentration, Z value and mosaicism can refer to the method of detecting fetal chromosomal aneuploidy abnormality in the present application, which will not be described in detail here.

Those skilled in the art can understand that all or part of the functions of the above method can be implemented in the form of hardware or in the form of a computer program. When all or part of the functions of the above method are implemented by a computer program, the program can be stored in a computer-readable storage medium. The storage medium may comprise: read-only memory, random access memory, magnetic disk, optical disk, hard disk, etc., and the above functions can be implemented by executing the program by a computer. For example, the program is stored in a memory of the device, and when the program in the memory is executed by the processor, all or part of the above functions can be implemented. In addition, when all or part of the functions in the above embodiments are implemented by a computer program, the program can also be stored in a storage medium such as a server, another computer, a magnetic disk, an optical disk, a flash disk or a mobile hard disk, and can be downloaded or copied and saved into the memory of the local device, or the system version of the local device can be updated, and when the program in the memory is executed by the processor, all or part of the functions of the above method can be implemented.

Therefore, based on the method of detecting fetal chromosomal aneuploidy abnormality in the present application, the present application proposes a device for detecting fetal chromosomal aneuploidy abnormality, which comprises a new Z-score calculation module and a fetal chromosomal aneuploidy abnormality determination module, the new Z value calculation module is configured to obtain a new Z value of a sample to be tested by calculation based on the fetal DNA concentration, Z value, and mosaicism in pregnant woman blood cell-free DNA; the mosaicism is a ratio of abnormal fetal cells to all fetal cells; the fetal chromosomal aneuploidy abnormality module is configured to determine whether aneuploidy abnormality occurs in the fetal chromosome of the sample to be tested on the basis of the new Z value .

In one embodiment of the present application, a device for detecting fetal chromosomal aneuploidy abnormality is shown in Figure 2, which comprises a data acquisition module **21,** a data processing module **22,** a mosaicism calculation module **23,** a model training module **24,** a new Z value calculation module **25** and a fetal chromosomal aneuploidy abnormality determination module **26.**

Wherein, the data acquisition module **21** is configured to acquire high-throughput sequencing data of pregnant woman blood cell-free DNA to be tested, for example, to acquire a fastq format file generated by the sequencer.

The data processing module **22** is configured to calculate the fetal DNA concentration, traditional Z value, average of corrected depths of each chromosome, and average of corrected depths of all autosomal chromosomes, for example, to calculate fetal DNA concentration, traditional Z value, average of corrected depths of each chromosome, average of corrected depths of all autosomal chromosomes, etc., by referring to the existing conventional NIPT protocol.

The mosaicism calculation module **23** is configured to calculate the mosaicism of each chromosome based on fetal DNA concentration.

For example, the mosaicism of each chromosome is calculated according to Formula I;

$$Mosaic_k = \frac{fra_k}{FF}$$

Formula I:

in Formula I, $Mosaic_k$ is the mosaicism of chromosome $k$, $fra_k$ is the relative fetal concentration of chromosome $k$, and $FF$ is the fetal DNA concentration;

$fra_k$ is calculated using Formula II;

$$fra_k = \frac{\overline{UR_k} - \overline{UR_{auto}}}{\overline{UR_{auto}}} * 2$$

Formula II:

in Formula II, $fra_k$ is the relative fetal concentration of chromosome $k$, $\overline{UR_k}$ is the average of corrected depths of chromosome $k$, and $\overline{UR_{auto}}$ is the average of corrected depths of all autosomal chromosomes;

in Formula I and Formula II, the value of $k$ ranges from 1 to 22;

when $Mosaic_k$ is 0, it indicates that the chromosome $k$ is normal; when $Mosaic_k$ is 1, it indicates that the chromosome $k$ of fetus is completely trisomic; when $Mosaic_k$ is between 0 and 1, it indicates that the chromosome $k$ of fetus is mosaic.

The model training module **24** is configured to use several samples with known fetal chromosome conditions as training samples (in which the training samples include positive samples and negative samples of fetal chromosomal aneuploidy abnormalities), to use fetal DNA concentration, Z value and mosaicism as inputs to train a machine learning model to obtain a model output value for characterizing fetal chromosome condition based on the integration of three variables of fetal DNA concentration, Z value and mosaicism, thereby obtaining a model, that is, the fetal chromosomal aneuploidy abnormality detection model; and it is configured, after the model is trained, to use positive samples to obtain the corresponding positive threshold, to use negative samples to obtain the corresponding negative threshold, and to use model output values of all negative samples to obtain the median thereof.

The new Z value calculation module **25** is configured to calculate the new Z value of the sample to be tested based on the fetal DNA concentration, Z value, and mosaicism in pregnant woman blood cell-free DNA of the sample to be tested; in which, the mosaicism is a ratio of fetal abnormal cells to all fetal cells.

For example, the new Z-value calculation module **25** comprises a model output value analysis sub-module and a Z-value mapping sub-module; the model output value analysis sub-module is configured to input the fetal DNA concentration, Z value and mosaicism of the sample to be tested into the fetal chromosomal aneuploidy abnormality detection model to obtain the model output value corresponding to the sample to be tested; the Z-value mapping sub-module is configured to obtain the new Z value of the sample to be tested by calculation based on the model output value of the sample to be tested, as well as the positive threshold, the negative threshold, the median of model output values of all negative samples; the positive threshold is the threshold of model output value corresponding to the positive sample, and the negative threshold is the threshold of model output value corresponding to the negative sample.

The fetal chromosomal aneuploidy abnormality determination module **26** is configured to determine whether aneuploidy abnormality occurs in the fetal chromosomes to be tested on the basis of the new Z value. For example, if the new Z value is greater than 3, it is determined as positive, that is, the fetal chromosomal aneuploidy abnormality occurs; if the new Z value is less than 1.96, it is judged as negative, that is, the fetal chromosome is normal.

Another embodiment of the present application further provides a device for detecting fetal chromosomal aneuploidy abnormality. The device comprises a memory and a processor; the memory is used to store a program; the processor is used to execute the program stored in the memory to implement the following method: obtaining the new Z value of the sample to be tested by calculation based on the fetal DNA concentration, Z value, and mosaicism in pregnant woman blood cell-free DNA of the sample to be tested, and determining whether aneuploidy abnormality occurs in the fetal chromosome of the sample to be tested on the basis of the new Z value; wherein, the mosaicism is a ratio of abnormal fetal cells to all fetal cells. Or, it is specifically used to implement the following method, comprising: a data acquisition step which comprises obtaining high-throughput sequencing data of pregnant women blood cell-free DNA to be tested; a data processing step which comprises calculating fetal DNA concentration and traditional Z value based on the obtained high-throughput sequencing data of pregnant women blood cell-free DNA to be tested; a mosaicism calculation step which comprises calculating the mosaicism of each chromosome based on the fetal DNA concentration; a model value analysis step which comprises inputting the fetal DNA concentration, traditional Z value and mosaicism into the fetal chromosomal aneuploidy abnormality detection model to obtain a model output value corresponding to the sample to be tested; a Z-value mapping step which comprises obtaining a new Z value by calculation based on the model output value of the sample to be tested, the positive threshold, the negative threshold, and the median of model output values of the negative samples; a fetal chromosomal aneuploidy abnormality determination step which comprises determining whether aneuploidy abnormality occurs in the chromosomes of the fetus to be tested on the basis of the new Z value.

Alternatively, the device comprises a memory and a processor; the memory is configured to store a program; the processor is configured to execute the program stored in the memory to implement the following method, comprising: using several samples of known fetal chromosome conditions as training samples, the training samples including positive and negative samples of fetal chromosomal aneuploidy abnormalities, using the fetal DNA concentration, Z value and mosaicism as inputs, performing machine learning model training to obtain a model output value for characterizing fetal chromosomal condition based on the integration of three variables of fetal DNA concentration, Z value and mosaicism, and thereby obtaining a model, that is, the fetal chromosomal aneuploidy abnormality detection model.

Another embodiment of the present application also provides a computer-readable storage medium. The storage medium comprises a program, and the program can be executed by a processor to implement the following method, comprising: obtaining a new Z value of the sample to be tested by calculation based on the fetal DNA concentration, Z value and mosaicism in pregnant women blood cell-free DNA of the sample to be tested, and determining whether aneuploidy abnormality occurs in the fetal chromosome of the sample to be tested on the basis of the new Z value; wherein, the mosaicism is a ratio of fetal abnormal cells to all fetal cells. Or, it is specifically used to implement the following method, comprising: a data acquisition step which comprises acquiring high-throughput sequencing data of pregnant women blood cell-free DNA to be tested; a data processing step which comprises calculating the fetal DNA concentration and traditional Z value based on the obtained high-throughput sequencing data of pregnant women blood cell-free DNA to be tested; a mosaicism calculation step which comprises calculating the mosaicism of each chromosome based on the fetal DNA concentration; a model value analysis step which comprises inputting the fetal DNA concentration, traditional Z value and mosaicism of the sample to be tested into the fetal chromosomal aneuploidy abnormality detection model to obtain a model output value corresponding to the sample to be tested; a Z value mapping step which comprises obtaining a new Z value by calculation based on the model output value of the sample to be tested, the positive threshold value, the negative threshold value, and the median of model output values of the negative samples; and a chromosome aneuploidy abnormality determination step which comprises determining whether aneuploidy abnormality occurs in the fetal chromosomes to be tested on the basis of the new Z value.

Alternatively, the storage medium comprises a program, and the program can be executed by a processor to implement the following method, comprising: using several samples with known fetal chromosomal conditions as training samples, the training samples including positive samples and negative samples of fetal chromosomal aneuploidy abnormalities, using the fetal DNA concentration, Z value and mosaicism as inputs to perform machine learning model training, obtaining a model output value for characterizing the fetal chromosome condition based on the integration of three variables of fetal DNA concentration, Z value and mosaicism, thereby obtaining a model, that is, the fetal chromosomal aneuploidy abnormality detection model.

The method and device of the present application are different from the existing technology in that:

(1) A unique detection indicator - mosaicism is created in the present application. Research has found that mosaicism has a good distinction degree between true positive and false positive samples that are currently reported only through the Z value (i.e. traditional Z value) method.

(2) In the present application, the three variables of fetal concentration, the unique indicator of the present application - mosaicism, and the traditional Z value are integrated, and in an embodiment, linear discriminant analysis (LDA) is specifically selected as the machine learning model to carry out the model training and the judgment of results. The study found that the judgment results of this model can reduce false positives and improve the detection effect as compared with the original results.

(3) The three variables used in the present application are all linear relationships. The linear relationship is simple and clear, avoiding the complexity caused by too many variables and different dimensions and distribution characteristics between variables. In one embodiment, the linear discriminant analysis (LDA) model is used for analysis. The model is simple and does not have the problem of overfitting.

(4) In the present application, a new Z-score conversion method is developed to convert values without statistical significance obtained by machine learning into a Z value that can be commonly used in clinic and comply with regulatory requirements, that is, the new Z-score of the present application. Moreover, the new Z value obtained through the Z value conversion method of the present application conforms to the normal distribution and can meet the current regulatory and clinical practices requirements.

(5) Comparing the new Z value of the present application with the traditional Z value, it can be found that the new Z value greatly reduces the volatility of data distribution, reduces the gray area and the number of retests, and improves the stability of detection results.

(6) In the machine learning-based solution provided by the present application, multiple variables are taken into account, and the real sample data accumulated by BGI are used for model training, so that the model can well learn and grasp the unique characteristics of the BGI's own data due to the factors such as experimental reagents and sequencing platforms, and thus can be better applied to the data currently produced by BGI. It can be understood that what the present application establishes is a set of learning methods for personalized data, and not only for BGI's own data.

The present application, a new detection indicator - mosaicism is first created, and the three variables of fetal DNA concentration, mosaicism and traditional Z value are further integrated to overcome the inaccuracy in the results of traditional NIPT that only relies on Z value to determine trisomy; in addition, a linear model is used to integrate the above three variables, the model is simple and does not have the problem of overfitting. Furthermore, the present application also developed a Z value conversion method, that is, Z value mapping, to convert the meaningless values obtained by the machine learning model into meaningful and clinically recognized Z value, namely $Z_{new}$, and the gray area and retests of the traditional Z value are reduced at the same time, and the stability of test results is improved as well.

It can be understood that on the basis of the present application, it is not ruled out that more parameters can be used for model training and fetal chromosomal aneuploidy abnormality analysis, for example, variables such as gestational age and maternal age, etc., can be taken into account. Of course, as the number of variables increases, the corresponding machine learning model also needs to be replaced, for example, a nonlinear QDA model may be used. In addition, the specific Z value segmented mapping in the present application can also be adjusted according to needs.

**Example 1**

In this example, the established fetal chromosomal aneuploidy abnormality detection model was used to predict samples with diagnostic results/follow-up results. Specifically, in this example, a total of 108,293 samples were used for model training. These samples were divided into negative and positive categories when entering model training, but they also contained 3 karyotypes, that was, the negative samples contained true negative and false positive samples, and the positive samples contained true positive samples. Since the fetal concentration calculation methods for male and female fetuses were different, the fetal concentration data characteristics of male and female fetuses were different. Since the fetal concentrations are one of the key variables of the model, two models were trained separately for male and female fetuses. The specific sample numbers were shown in Table 1.

Table 1: Samples used for model training

|  | Male | Female |
|---|---|---|
| True positive | 798 | 620 |
| False positive | 234 | 318 |
| True negative | 56864 | 49459 |
| Total | 57896 | 50397 |

Table 2: Examples of sample data used for model training

|  | Fetal concentration | Traditional Z value | Mosaicism |
|---|---|---|---|
| True positive 1 | 0.149 | 14.501 | 0.900 |
| True positive 2 | 0.120 | 10.058 | 0.885 |
| False positive 1 | 0.293 | 4.834 | 0.173 |
| False positive 2 | 0.389 | 6.821 | 0.158 |
| True negative 1 | 0.229 | -0.810 | -0.035 |

(continued)

|  | Fetal concentration | Traditional Z value | Mosaicism |
|---|---|---|---|
| True negative 2 | 0.120 | -0.596 | -0.049 |

In this example, the fetal DNA concentration, traditional Z value and mosaicism of the training samples, as shown in Table 2, were input into the LDA model for training and the model output values were obtained. Taking the medians of the values obtained by machine learning of the negative samples, we obtain the "medians of the model output values", which were the *Med* in the subsequent mapping formula. The medians obtained by calculation in this example were shown in Table 3.

Table 3: Medians of model output values

|  | Male | Female |
|---|---|---|
| Median of T13 model output values | -0.281 | -0.194 |
| Median of T18 model output values | -0.242 | -0.166 |
| Median of T21 model output values | -0.263 | -0.203 |

Before mapping, by artificially observing the distribution of true negative, false positive and true positive samples, the thresholds for the LD values were defined so that: 1) no true positive sample would be judged as negative; 2) as many true positive samples as possible would be judged as positive; 3) as few false positive samples as possible were judged as positive. The thresholds of LD values were determined according to the above principles, which were the positive threshold ($cut_p$) and negative threshold ($cut_n$) in the mapping formula. The specific values in this example were shown in Table 4.

Table 4: Thresholds for LD values

|  | Male | | Female | |
|---|---|---|---|---|
|  | $cut_n$ | $cut_p$ | $cut_n$ | $cut_p$ |
| T13 | 3.26 | 4.50 | 1.26 | 3.50 |
| T18 | 1.74 | 3.25 | 2.26 | 3.00 |
| T21 | 1.44 | 3.00 | 1.48 | 3.00 |

After mapping, the commonly used clinical values 1.96 and 3 were used as the thresholds of the new Z value, and the following mapping method was obtained:

when the model output value of the sample to be tested was greater than the positive threshold,

$$Z_{new}=LD-cut_p+3;$$

when the model output value of the sample to be tested was less than the positive threshold and greater than the negative threshold,

$$Z_{new}=\frac{LD-cut_n}{cut_p-cut_n}*1.04+1.96;$$

when the model output value of the sample to be tested was less than the negative threshold,

$$Z_{new}=\frac{LD-Med}{cut_n-Med}*1.96;$$

in the above formula, $Z_{new}$ is the new Z value, *LD* was the model output value of the sample to be tested, $cut_p$ was the positive threshold, $cut_n$ was the negative threshold, and *Med* was the median of the model output values of all negative samples.

A total of 10,240 samples tested by BGI in actual clinical practicess and underwent prenatal diagnosis/postnatal follow-up were selected. The test results of these samples were given based on the traditional Z values in actual clinical testing, and subsequent prenatal diagnosis/postnatal follow-up was carried out based on the test results. Therefore, each of the samples could be classified into three categories: true positive, false positive, and true negative, according to the test results and the results of prenatal diagnosis/postnatal follow-up of each sample. The specific information of the samples was shown in Table 5.

Wherein, the traditional Z value calculation method was as follows:

$$Z_i = \text{mean}\left(\frac{\overline{UR_i} - \overline{UR_j}}{\sqrt{\frac{SD_i^2}{L_i} + \frac{SD_j^2}{L_j}}}\right)$$

(j=1...22, j≠i, two values before and after it were removed after sorting)
wherein:

$\overline{UR_i}$: mean value of UR of chromosome $i$;
$\overline{UR_j}$: mean value of UR of chromosome $j$;
$SD_i$: represented the standard deviation of UR of chromosome $i$;
$SD_j$: represented the standard deviation of UR of chromosome $j$;
$L_i$: represented the number of windows divided for chromosome $i$;
$L_j$: represented the number of windows divided for chromosome $j$;
$Z_i$: represented the significance of aneuploidy of chromosome i, indicating the difference from euploidy.

Table 5: Trisomy detection results given by traditional Z values

|  | T21 | T18 | T13 |
|---|---|---|---|
| True positive | 87 | 45 | 22 |
| False positive | 14 | 33 | 39 |
| PPV | 0.86 | 0.58 | 0.36 |
| True negative | 10000 | | |

Table 6: Examples of sample data used for model testing

|  | Fetal concentration | Traditional Z value | Mosaicism |
|---|---|---|---|
| True positive 1 | 0.067 | 5.824 | 0.885 |
| True positive 2 | 0.146 | 11.714 | 0.808 |
| False positive 1 | 0.188 | 3.602 | 0.205 |
| False positive 2 | 0.187 | 4.186 | 0.252 |
| True negative 1 | 0.115 | 1.137 | 0.090 |
| True negative 2 | 0.059 | -1.125 | -0.192 |

It could be seen that the testing based on traditional Z values showed that the positive predictive values of T21, T18, and T13 were 0.86, 0.58, and 0.36, respectively, and the problem of false positives was relatively prominent.

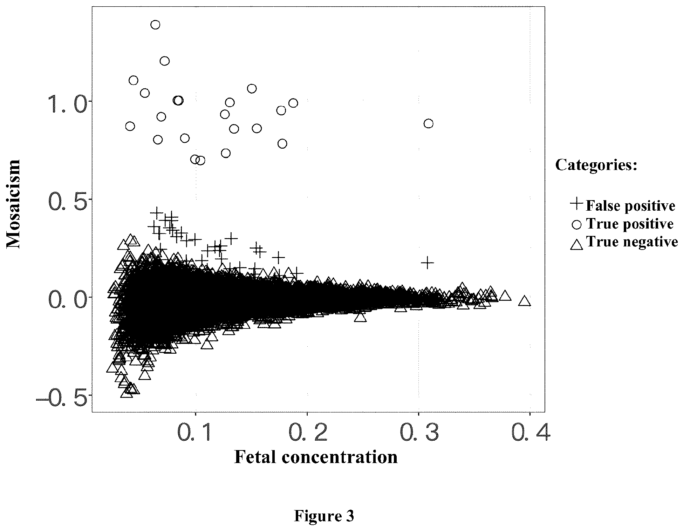

Using the fetal chromosomal aneuploidy abnormality detection model and the method for detecting fetal chromosomal aneuploidy abnormality in the present application, the mosaicism values of the above 10,240 samples were calculated. Taking T13 as an example, the results were shown in Figure 3, in which true positive, false positive and true negative samples could be well distinguished based on the mosaicism values. Further, the three variables of mosaicism, fetal concentration, and traditional Z value were input into the trained machine learning model, and then a new Z value was generated through Z value mapping. Some sample data used for model testing were shown in Table 6. The above 10,240 samples were re-judged based on the new Z values. Z>3 was judged as positive and Z<1.96 was judged as negative, thereby generating new test results, which were shown in Table 7.

Table 7: Trisomy detection results given by the improved fetal chromosomal aneuploidy abnormality detection method

|  | T21 | T18 | T13 |
|---|---|---|---|
| True positive | 101 | 78 | 61 |
| False positive | 0 | 0 | 0 |
| PPV | 1.00 | 1.00 | 1.00 |
| True negative | 10000 | | |

The results in Table 7 show that using the new Z value, 14 cases of T21 false positives, 33 cases of T18 false positives, and 39 cases of T13 false positives were all correctly judged as negative. At the same time, 87 cases of T21 true positives, 45 cases of T18 true positives, and 22 cases of T18 true positives were correctly judged as negative. T13 true positives and 10,000 true negative samples can still be determined correctly. Therefore, the positive predictive values of T21, T18, and T13 all reach 100%, the sensitivity is 100%, and the specificity is 100%. This ensures sensitivity and greatly reduces false positives in the test., improve PPV and specificity.

Example 2

In this example, the established model was used to detect continuous samples from the production line.

Due to factors such as the collection of diagnosis/follow-up results, the samples with karyotype were not continuous samples from a single center. Therefore, their data distribution characteristics could not reflect the true distribution characteristics of the population, and thus the true distribution characteristics of the new Z value could not be evaluated. Hence, using the continuous samples received by a certain medical laboratory of BGI over a period of time, the distribution characteristics of the new Z values obtained were evaluated, and they were compared with the traditional Z values to demonstrate the real characteristics and laws of the new Z values in actual practices.

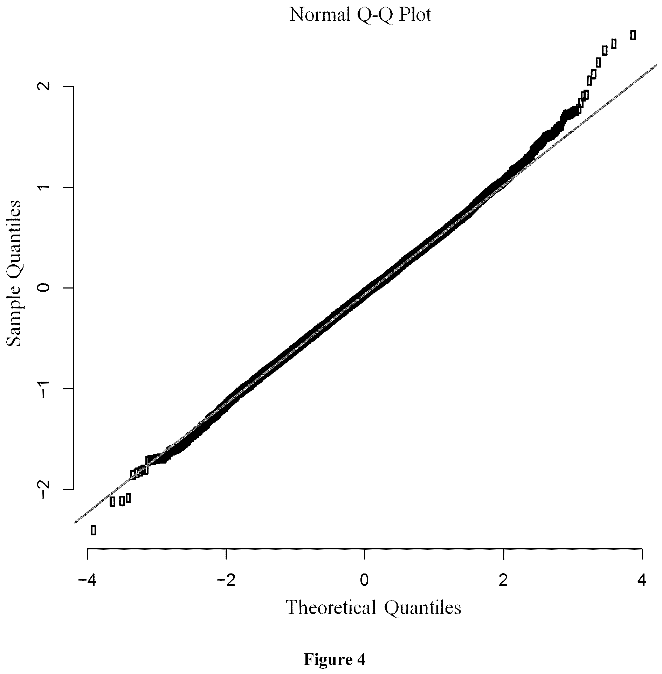

10,000 consecutive samples that were clinically tested at a single medical laboratory of BGI within a certain period of time were selected, and the fetal chromosomal aneuploidy abnormality detection model and fetal chromosomal aneuploidy abnormality detection method of the present application were used to calculate the new Z values for these 10,000 samples. Taking the Z values of chromosome 21 as example, and it was checked whether the distribution of the Z values of chromosome 21 conformed to the normal distribution, and the results were shown in Figure 4. The results in Figure 4 showed that the new Z values of 10,000 samples in a single center and in a continuous time period were basically located on the diagonal line of the Q-Q diagram, in which individual samples that deviate more from the diagonal line of the Q-Q diagram were positive samples with stronger signals, and Figure 4 showed that the new Z values had very good normality.

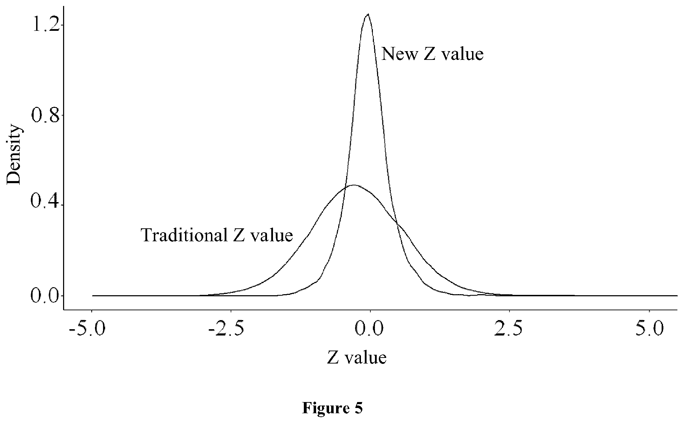

To further compare the distribution of the new Z values with the traditional Z values, the Z values of chromosome 13 were taken as example, which were shown in Figure 5. The results in Figure 5 showed that, first of all, the center of the new Z value distribution was closer to 0, indicating that the new Z values were more consistent with the normal distribution centered at 0 as compared to the traditional Z values; secondly, the new Z value distribution was more concentrated than the traditional Z values, indicating that the new Z values had lower volatility and better stability than the traditional Z values.

The new Z values had smaller fluctuations in comparison with the traditional Z values, which could bring about the effect of reducing the gray area rate. In this example, it was further demonstrated with a larger sample size. Specifically, 360,786 clinical samples tested by a single medical laboratory of BGI in 2020 were taken. These samples were tested a total of 383,306 times. The new Z values produced T21 gray area for 785 times, T18 gray area for 345 times, and T13 gray area for 288 times in the 383,306 tests. The gray area rates of T21, T18, and T13 were 0.22%, 0.09%, and 0.08%, respectively; and the overall gray area rate of trisomy testing was 0.39%. In comparison, the traditional Z value produced T21 gray area for 3071 times, T18 gray area for 4350 times, and T13 gray area for 2335 times, the gray area rates of T21, T18, and T13 were 0.80%, 1.14%, and 0.61% respectively; and the overall gray area rate of trisomy testing was 2.55%, as shown in Table 8.

Table 8: Comparison results of gray area sample number and gray area rate between traditional Z value and new Z value

| | Original results | | Judgment results based on new Z values | |
| --- | --- | --- | --- | --- |
| | Gray area samples | Gray area rate | Gray area samples | Gray area rate |
| T21 | 3071 | 0.80% | 785 | 0.22% |
| T18 | 4350 | 1.14% | 345 | 0.09% |
| T13 | 2335 | 0.61% | 288 | 0.08% |
| Total | 9756 | 2.25% | 1418 | 0.39% |

The results in Table 8 showed that the new Z values generated by the method of the present application could reduce the gray area rate of trisomy detection to about one-tenth of the previous one, which significantly reduced retests caused by gray areas, thereby improving the detection performance of NIPT.

The above contents are further detailed descriptions of the present application in combination with specific embodiments, and it cannot be concluded that the specific embodiments of the present application are limited to these descriptions. For those of ordinary skill in the technical field to which the present application belongs, several simple deductions or substitutions can be made without departing from the concept of the present application.

**Claims**

1. A method for detecting fetal chromosomal aneuploidy abnormality, **characterized by**: comprising obtaining a new Z value of a sample to be tested by calculation based on the fetal DNA concentration, Z value and mosaicism in cell-free DNA of a pregnant woman blood sample to be tested, and determining whether an aneuploidy abnormality occurs in the fetal chromosome of the sample to be tested;
   wherein the mosaicism is a ratio of abnormal fetal cells to all fetal cells.

2. The method according to claim 1, **characterized in that**: the new Z value of the sample to be tested is obtained by calculation based on the fetal DNA concentration, Z value, and mosaicism in the cell-free DNA of the pregnant woman blood sample to be tested, which comprises inputting the fetal DNA concentration, Z value and mosaicism into a fetal chromosomal aneuploidy abnormality detection model to obtain a model output value corresponding to the sample to be tested, and mapping the model output value to obtain the new Z value of the sample to be tested;

   the fetal chromosomal aneuploidy abnormality detection model is a model obtained by using several samples with known fetal chromosomal conditions as training samples, the training samples including positive samples and negative samples of fetal chromosomal aneuploidy abnormalities, and using the fetal DNA concentration, Z value and mosaicism as inputs to train a machine learning model to obtain a model output value for characterizing fetal chromosome condition based on the integration of three variables of fetal DNA concentration, Z value, and mosaicism;
   preferably, the new Z value of the sample to be tested is obtained by mapping the model output value, which comprises obtaining the new Z value of the sample to be tested by calculation based on the model output value of the sample to be tested, a positive threshold, a negative threshold, and a median of model output values of all negative samples;
   the positive threshold is a threshold of model output values corresponding to positive samples, and the negative threshold is a threshold of model output values corresponding to negative samples;
   preferably, the median of model output values of all negative samples is a median of model output values of all negative samples obtained by re-inputting all negative training samples into the fetal chromosomal aneuploidy abnormality detection model;
   preferably, the new Z value of the sample to be tested is obtained by mapping the model output value, comprising the following mapping method:

   when the model output value of the sample to be tested is greater than the positive threshold,

   $$Z_{new} = LD - cut_p + 3;$$

when the model output value of the sample to be tested is less than the positive threshold and greater than the negative threshold,

$$Z_{new=} \frac{LD - cut_n}{cut_p - cut_n} * 1.04 + 1.96 \quad ;$$

when the model output value of the sample to be tested is less than the negative threshold,

$$Z_{new=} \frac{LD - Med}{cut_n - Med} * 1.96 \quad ;$$

in the above formula, $Z_{new}$ is the new Z value, $LD$ is the model output value of the sample to be tested, $cut_p$ is the positive threshold, $cut_n$ is the negative threshold, and $Med$ is the median of model output values of all negative samples;

preferably, whether an aneuploidy abnormality occurs in the fetal chromosomes of the sample to be tested is determined based on the new Z value, which comprises if the new Z value is greater than 3, it is determined to be positive, that is, the fetal chromosomal aneuploidy abnormality occurs; if the new Z value is less than 1.96, it is determined as negative, that is, the fetal chromosomes are normal;

preferably, the machine learning model is a linear discriminant analysis model;

preferably, the fetal abnormal cells are cells containing fetal chromosomal aneuploidy abnormalities;

preferably, the fetal DNA concentration and Z value in the pregnant women blood cell-free DNA are obtained by calculation through high-throughput sequencing data of the pregnant women blood cell-free DNA.

3. The method according to claim 1 or 2, **characterized in that**: the mosaicism is calculated by Formula I;

$$Mosaic_k = \frac{fra_k}{FF}$$
Formula I

in Formula I, $Mosaic_k$ is the mosaicism of chromosome $k$, $fra_k$ is the relative fetal concentration of chromosome $k$, and $FF$ is the fetal DNA concentration;

$fra_k$ is calculated using Formula II;

$$fra_k = \frac{\overline{UR_k} - \overline{UR_{auto}}}{\overline{UR_{auto}}} * 2$$
Formula II

in Formula II, $fra_k$ is the relative fetal concentration of chromosome $k$, $\overline{UR_k}$ is the average of corrected depths of chromosome $k$, and $\overline{UR_{auto}}$ is the average of corrected depths of all autosomal chromosomes;

in Formula I and Formula II, the value of $k$ ranges from 1 to 22;

when $Mosaic_k$ is 0, it indicates that the chromosome $k$ of fetus is normal; when $Mosaic_k$ is 1, it indicates that the chromosome $k$ of fetus is completely trisomic; when $Mosaic_k$ is between 0 and 1, it indicates that the chromosome $k$ of fetus is mosaic;

preferably, the average value of corrected depth of each chromosome and the average value of corrected depth of all autosomal chromosomes are calculated and obtained through high-throughput sequencing data of the pregnant women blood cell-free DNA.

4. A construction method for a fetal chromosomal aneuploidy abnormality detection model, **characterized by**: comprising using several samples with known fetal chromosomal conditions as training samples, the training samples including positive samples and negative samples of fetal chromosomal aneuploidy abnormalities, using the fetal DNA concentration, Z value and mosaicism as inputs to train a machine learning model to obtain a model output value for characterizing fetal chromosome condition based on the integration of three variables of fetal DNA concentration, Z value and mosaicism, thereby obtain a model, that is, the fetal chromosomal aneuploidy abnormality detection model.

5. The construction method according to claim 4, **characterized in that**: the fetal DNA concentration and Z value are obtained by calculation according to high-throughput sequencing data of pregnant women blood cell-free DNA; the mosaicism is a ratio of abnormal fetal cells to all fetal cells;

preferably, the fetal abnormal cells are cells containing fetal chromosomal aneuploidy abnormalities;
preferably, the mosaicism is calculated by Formula I;

$$Mosaic_k = \frac{fra_k}{FF}$$
Formula I

in Formula I, $Mosaic_k$ is the mosaicism of chromosome $k$, $fra_k$ is the relative fetal concentration of chromosome $k$, and $FF$ is the fetal DNA concentration;
$fra_k$ is calculated using Formula II;

$$fra_k = \frac{\overline{UR}_k - \overline{UR}_{auto}}{\overline{UR}_{auto}} * 2$$
Formula II

in Formula II, $fra_k$ is the relative fetal concentration of chromosome $k$, $\overline{UR}_k$ is the average of corrected depths of chromosome $k$, and $\overline{UR}_{auto}$ is the average of corrected depths of all autosomal chromosomes;
in Formula I and Formula II, the value of $k$ ranges from 1 to 22;
when $Mosaic_k$ is 0, it indicates that the chromosome $k$ of fetus is normal; when $Mosaic_k$ is 1, it indicates that the chromosome $k$ of fetus is completely trisomic; when $Mosaic_k$ is between 0 and 1, it indicates that the chromosome $k$ of fetus is mosaic;
preferably, the average value of corrected depth of each chromosome and the average value of corrected depth of all autosomal chromosomes are calculated and obtained through high-throughput sequencing data of the pregnant women blood cell-free DNA;
preferably, the machine learning model is a linear discriminant analysis model.

6. A device for detecting fetal chromosomal aneuploidy abnormality, **characterized by**: comprising a new Z-score calculation module and a fetal chromosomal aneuploidy abnormality determination module;

the new Z value calculation module is configured to obtain a new Z value of a sample to be tested on the basis of fetal DNA concentration, Z value and mosaicism in pregnant woman blood cell-free DNA of the sample to be tested; the mosaicism is a ratio of fetal abnormal cells to all fetal cells;
the fetal chromosomal aneuploidy abnormality module is configured to determine whether an aneuploidy abnormality occurs in the fetal chromosome of the sample to be tested on the basis of the new Z value.

7. The device according to claim 6, **characterized in that**: the new Z value calculation module is further configured to input the fetal DNA concentration, Z value and mosaicism into a fetal chromosomal aneuploidy abnormality detection model to obtain a model output value corresponding to the sample to be tested, and to obtain the new Z value of the sample to be tested by mapping the model output value;
the fetal chromosomal aneuploidy abnormality detection model is a model obtained by using several samples with known fetal chromosomal conditions as training samples, the training samples including positive samples and negative samples of fetal chromosomal aneuploidy abnormalities, and using the fetal DNA concentration, Z value and mosaicism as inputs to train a machine learning model; the model output value is used to characterize the fetal chromosome condition by integrating the three variables of fetal DNA concentration, Z value, and mosaicism.

8. The device according to claim 7, further comprising a model training module, in which several samples of known fetal chromosome conditions are used as training samples, the training samples include positive samples and negative samples of fetal chromosomal aneuploidy abnormalities, and the fetal DNA concentration, Z value and mosaicism are used as inputs to train a machine learning model to obtain a model output value to characterize the fetal chromosome situation based on the integration of three variables of fetal DNA concentration, Z value and mosaicism, thereby obtaining a model, that is, the fetal chromosomal aneuploidy abnormality detection model;

preferably, the machine learning model is a linear discriminant analysis model;
preferably, the new Z value calculation module comprises a model output value analysis sub-module and a Z value mapping sub-module; the model output value analysis sub-module is configured to input the fetal DNA concentration, Z value and mosaicism of the sample to be tested input into the fetal chromosomal aneuploidy abnormality detection model to obtain the model output value corresponding to the sample to be tested; the Z value mapping sub-module is configured to obtain the new Z value of the sample to be tested by calculation based on the model output value of the sample to be tested, as well as a positive threshold, a negative threshold, and a median of model output values of all negative samples; the positive threshold is a threshold of model output values corresponding to the positive samples, and the negative threshold is a threshold of model output values corresponding to the negative samples;

preferably, the Z value mapping sub-module is configured to obtain the new Z value according to the following method:

when the model output value of the sample to be tested is greater than the positive threshold,

$$Z_{new}=LD\text{-}cut_p+3;$$

when the model output value of the sample to be tested is less than the positive threshold and greater than the negative threshold,

$$Z_{new=}\frac{\frac{LD-cut_n}{cut_p-cut_n}*1.04+1.96}{};$$

when the model output value of the sample to be tested is less than the negative threshold,

$$Z_{new=}\frac{\frac{LD-Med}{cut_n-Med}*1.96}{};$$

in the above formula, $Z_{new}$ is the new Z value, $LD$ is the model output value of the sample to be tested, $cut_p$ is the positive threshold, $cut_n$ is the negative threshold, and $Med$ is the median of model output values of all negative samples;

preferably, whether an aneuploidy abnormality occurs in the fetal chromosomes of the sample to be tested is determined based on the new Z value, which comprises if the new Z value is greater than 3, it is determined to be positive, that is, the fetal chromosomal aneuploidy abnormality occurs; if the new Z value is less than 1.96, it is determined as negative, that is, the fetal chromosomes are normal.

9. The device according to claim 6, **characterized by**: further comprising a data acquisition module for acquiring high-throughput sequencing data of pregnant women blood cell-free DNA of the sample to be tested;

preferably, further comprising a data processing module for calculating the fetal DNA concentration and Z value based on the obtained high-throughput sequencing data of pregnant women blood cell-free DNA;

preferably, the data processing module is configured to calculate the average of corrected depths of each chromosome and the average of corrected depths of all autosomal chromosomes based on the obtained high-throughput sequencing data of pregnant women blood cell-free DNA to be tested;

preferably, further comprising a mosaicism calculation module for calculating the mosaicism of each chromosome according to Formula I;

$$Mosaic_k = \frac{fra_k}{FF}$$

Formula I

in Formula I, $Mosaic_k$ is the mosaicism of chromosome $k$, $fra_k$ is the relative fetal concentration of chromosome $k$, and $FF$ is the fetal DNA concentration;

$fra_k$ is calculated using Formula II;

$$fra_k = \frac{\overline{UR}_k - \overline{UR}_{auto}}{\overline{UR}_{auto}} * 2$$

Formula II

in Formula II, $fra_k$ is the relative fetal concentration of chromosome $k$, $\overline{UR}_k$ is the average of corrected depths of chromosome $k$, and $\overline{UR}_{auto}$ is the average of corrected depths of all autosomal chromosomes;

in Formula I and Formula II, the value of $k$ ranges from 1 to 22;

when $Mosaic_k$ is 0, it indicates that the chromosome $k$ of fetus is normal; when $Mosaic_k$ is 1, it indicates that the chromosome $k$ of fetus is completely trisomic; when $Mosaic_k$ is between 0 and 1, it indicates that the chromosome $k$ of fetus is mosaic.

10. A device for detecting fetal chromosomal aneuploidy abnormality, **characterized in that** the device comprises:

a memory, which is used to store a program;

a processor, which is configured to implement the method for detecting fetal chromosomal aneuploidy abnormality according to any one of claims 1 to 3 or the construction method for fetal chromosomal aneuploidy detection model according to claim 4 or 5 by executing the program stored in the memory.

11. A computer-readable storage medium, **characterized by**: comprising a program, which is configured to be executed by a processor to implement the method for detecting fetal chromosomal aneuploidy abnormality according to any one of claims 1 to 3 or the construction method for fetal chromosomal aneuploidy abnormality detection model according to claim 4 or 5.

11 — Step of acquiring data

12 — Step of processing data

13 — Step of calculating mosaicism

14 — Step of calculating new Z value

15 — Step of determining fetal chromosomal aneuploidy abnormality

Figure 1

21 — Data acquisition module

22 — Data processing module

23 — Mosaicism calculation module

24 — Model training module

25 — New Z value calculation module

26 — Fetal chromosomal aneuploidy abnormality determination module

Figure 2

Figure 3

Normal Q-Q Plot

Figure 4

**Figure 5**

## INTERNATIONAL SEARCH REPORT

| International application No. |
| --- |
| **PCT/CN2023/080510** |

### A. CLASSIFICATION OF SUBJECT MATTER

G16B20/20(2019.01)i;G16B40/00(2019.01)i;G16B30/00(2019.01)i

According to International Patent Classification (IPC) or to both national classification and IPC

### B. FIELDS SEARCHED

Minimum documentation searched (classification system followed by classification symbols)

IPC: G16B20/-; G16B40/-; G16B30/-

Documentation searched other than minimum documentation to the extent that such documents are included in the fields searched

Electronic data base consulted during the international search (name of data base and, where practicable, search terms used)

CNABS, CNTXT, ENTXTC, CNKI: z分数, z值, 浓度, 嵌合, 染色体, 非整倍体, 训练, 样本, 异常, 异常细胞, 占比, DNA浓度; ENTXT, 必应检索 BING, 百度学术 BAIDU SCHOLAR, web of science: chromosomal abnormalities, chimeric, DNA, concentration, z-score

### C. DOCUMENTS CONSIDERED TO BE RELEVANT

| Category* | Citation of document, with indication, where appropriate, of the relevant passages | Relevant to claim No. |
| --- | --- | --- |
| PX | CN 115223654 A (SHENZHEN BGI GENOMICS CO., LTD.) 21 October 2022 (2022-10-21) claims 1-11 | 1-11 |
| Y | YANG, Jianfeng et al. "Improving the calling of non-invasive prenatal testing on 13-/18-/21-trisomy by support vector machine discrimination" *PLOS ONE, doi:10.1371/journal.pone.0207840*, Vol. 13, No. 12, 05 December 2018 (2018-12-05), abstract, page 6SVM discrimination, page 8Other discrimination methods | 1, 4, 6-7, 10-11 |
| Y | 许旭平 (XU, Xuping). "母体血浆中胎儿游离DNA定量及其在无创产前筛查中的应用研究 (Methods to Quantify Cell-free Fetal DNA Fraction in Maternal Plasma: Its Application in Non-invasive Prenatal Chromosomal Aneuploidy Detection Using Next Generation Sequencing)" *中国优秀硕士学位论文全文数据库 医药卫生辑 (Medicine & Public Health, China Master's Theses Full-Text Database)*, No. 2019(01), 15 January 2019 (2019-01-15), page 37, paragraph 3 | 1, 4, 6-7, 10-11 |

✓ Further documents are listed in the continuation of Box C.  ✓ See patent family annex.

| * | Special categories of cited documents: |
| --- | --- |
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
| --- | --- |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
| --- | --- |
| **21 June 2023** | **26 June 2023** |

| Name and mailing address of the ISA/CN | Authorized officer |
| --- | --- |
| **China National Intellectual Property Administration (ISA/CN)** **China No. 6, Xitucheng Road, Jimenqiao, Haidian District, Beijing 100088** | |
| | Telephone No. |

Form PCT/ISA/210 (second sheet) (July 2022)

**INTERNATIONAL SEARCH REPORT**

| International application No. |
| --- |
| **PCT/CN2023/080510** |

**C.     DOCUMENTS CONSIDERED TO BE RELEVANT**

| Category* | Citation of document, with indication, where appropriate, of the relevant passages | Relevant to claim No. |
| --- | --- | --- |
| Y | CN 112669901 A (BEIJING USCI MEDICAL LABORATORY CO., LTD.) 16 April 2021 (2021-04-16) <br> description, paragraph 118 | 1, 4, 6-7, 10-11 |
| A | CN 107133495 A (BEIJING USCI BIOTECHNOLOGY CO., LTD. et al.) 05 September 2017 (2017-09-05) <br> entire document | 1-11 |
| A | WO 2017009372 A2 (CARTAGENIA N.V.) 19 January 2017 (2017-01-19) <br> entire document | 1-11 |

Form PCT/ISA/210 (second sheet) (July 2022)

**INTERNATIONAL SEARCH REPORT**
Information on patent family members

International application No.

**PCT/CN2023/080510**

| Patent document cited in search report | | | Publication date (day/month/year) | Patent family member(s) | | | Publication date (day/month/year) |
|---|---|---|---|---|---|---|---|
| CN | 115223654 | A | 21 October 2022 | None | | | |
| CN | 112669901 | A | 16 April 2021 | None | | | |
| CN | 107133495 | A | 05 September 2017 | CN | 107133495 | B | 13 July 2018 |
| WO | 2017009372 | A2 | 19 January 2017 | EP | 3636777 | A1 | 15 April 2020 |
| | | | | US | 2018211002 | A1 | 26 July 2018 |
| | | | | AU | 2016293025 | A1 | 02 November 2017 |
| | | | | EP | 3322816 | B1 | 01 January 2020 |

Form PCT/ISA/210 (patent family annex) (July 2022)